Europäisches Patentamt

(19) European Patent Office

Office européen des brevets

(11) Veröffentlichungsnummer: **0 064 714**
**B1**

(12) **EUROPÄISCHE PATENTSCHRIFT**

(45) Veröffentlichungstag der Patentschrift:
**21.08.85**

(51) Int. Cl.⁴: **C 07 D 249/18**, C 07 D 253/08, C 06 B 23/00, C 07 K 5/10, C 07 K 7/06

(21) Anmeldenummer: **82103768.6**

(22) Anmeldetag: **03.05.82**

(54) Verfahren zur Phlegmatisierung von Hydroxy-benzotriazolen und Hydroxy-oxo-dihydro-benzotriazinen.

(30) Priorität: **07.05.81 DE 3117985**

(43) Veröffentlichungstag der Anmeldung:
**17.11.82 Patentblatt 82/46**

(45) Bekanntmachung des Hinweises auf die Patenterteilung:
**21.08.85 Patentblatt 85/34**

(84) Benannte Vertragsstaaten:
**DE**

(56) Entgegenhaltungen:
**DE - A - 1 939 187**
**DE - A - 2 455 623**

**R. MEYER: "Explosivstoffe:, 3. Auflage 1973, Verlag Chemie, Weinheim, DE.**
**METHODEN DER ORGANISCHEN CHEMIE, (HOUBEN WEYL), Auflage IV, Band 1, Teil 2, 1959, Georg Thieme Verlag, Stuttgart, DE.**

(73) Patentinhaber: **HOECHST AKTIENGESELLSCHAFT, Postfach 80 03 20, D-6230 Frankfurt am Main 80 (DE)**

(72) Erfinder: **Jäger, Georg, Dr., Am Rehsteig 9, D-6232 Bad Soden am Taunus (DE)**
Erfinder: **Radscheit, Kurt, Dr., Johann-Strauss-Strasse 20, D-6233 Kelkheim (Taunus) (DE)**
Erfinder: **Grewer, Theodor, Dr., Humperdinckweg 18, D-6232 Bad Soden am Taunus (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Gegenstand der Erfindung ist ein Verfahren zur Phlegmatisierung von Hydroxy-benzotriazolen und Hydroxy-oxo-dihydrobenzotriazinen.

Hydroxy-benzotriazole und Hydroxy-oxo-dihydro-benzotriazine werden unter anderem zur Herabsetzung der Racemisierung bei Peptidsynthesen eingesetzt, insbesondere bei Synthesen nach der Dicyclohexylcarbodiimid- oder Aktivester-Methode.

Es hat sich gezeigt, daß sowohl Hydroxy-benzotriazole als auch Hydroxy-oxo-dihydro-benzotriazine äußerst explosionsgefährliche Substanzen sind. Bei ihrer Herstellung, Trocknung, Zerkleinerung durch Mahlen oder Sieben, Lagerung (insbesondere in der Nähe von Heizkörpern) und auch bei ihrem Einsatz in chemischen Reaktionen sind Unfälle schwerster Art zu befürchten. Bei Ausbruch eines Feuers kann es zu Explosionen kommen. Aufgrund der Ergebnisse von Schlagempfindlichkeits- und Stahlhülsentests (sogenannter Koenen-Test) handelt es sich bei diesen Substanzen um Explosivstoffe im Sinne der einschlägigen Gesetze. Demgemäß muß ihre Handhabung und insbesondere ihre Lagerung besonderen Vorschriften unterliegen. Die praktische Anwendung von Hydroxy-benzotriazole und Hydroxy-oxo-dihydro-benzotriazine wird durch diese Eigenschaften stark eingeschränkt oder gar unmöglich gemacht.

Durch Zusatz von Wasser oder anderen nicht-brennbaren Flüssigkeiten kann die Explosionsgefährlichkeit nicht beseitigt werden. So erwies sich in eigenen Versuchen ein Gemisch aus 40 Gewichtsteilen 3-Hydroxy-4-oxo-3,4-dihydro-1,2,3-benzotriazin und 60 Gewichtsteilen Wasser immer noch explosionsgefährlich (vgl. Tabelle). Es wurde aber überraschend gefunden, daß durch Bemischung von feinkörnigen Feststoffen die Explosionsgefährlichkeit der Hydroxy-benzotriazole und der Hydroxy-oxo-dihydro-benzotriazine, insbesondere wenn sie in fester Form in Substanzen vorliegen, stark herabgesetzt oder sogar beseitigt werden kann.

Gegenstand der Erfindung ist demgemäß ein Verfahren zu Phlegmatisierung von Hydroxy-benzotriazolen oder Hydroxy-oxo-dihydro-benzotriazinen, das dadurch gekennzeichnet ist, daß man diesen feinverteilte inerte Feststoffe zusetzt.

Durch diesen Zusatz entstehen Gemische, die nicht mehr, wie die unverschnittenen Hydroxy-benzotriazole oder Hydroxy-oxo-dihydro-benzotriazine explosionsgefährlich sind.

Gegenstand der Erfindung sind daher weiterhin (A) ein Hydroxy-benzotriazol oder ein Hydroxy-oxo-dihydro-benzotriazin und (B) einen feinverteilten inerten Feststoff enthaltende Mittel.

Als inerte Feststoffe kommen Substanzen in Frage, die mit den Hydroxy-benzotriazolen bzw. Hydroxy-oxo-dihydro-benzotriazinen keine chemische Reaktionen eingehen und die auch die Reaktionen, in denen die Hydroxy-benzotriazole bzw. Hydroxy-oxo-dihydro-benzotriazine mitwirken, nicht stören. In diesem Sinne kann beispielsweise, wenn die Hydroxy-benzotriazole oder Hydroxy-oxo-dihydro-benzotriazine in Peptidsynthesen eingesetzt werden, Dicyclohexylharnstoff als Phlegmatisierungsmittel benutzt werden, da dieser bei den Kupplungsreaktionen als Nebenprodukt ohnehin entsteht und wie üblich durch Filtration entfernt werden kann. Es können aber auch andere inerte Feststoffe, z. B. Filterhilfsmittel auf Silikatbasis, Aktivkohle, Steinmehl, zugesetzt werden. Weiterhin kann man die Hydroxy-benzotriazole oder Hydroxy-oxo-dihydro-benzotriazine mit Ausgangssubstanzen mischen, die in der Reaktion eingesetzt werden.

Die als Phlegmatisierungsmittel einzusetzenden Feststoffe müssen in feinverteilter Form vorliegen. Die Teilchengröße spielt hierbei keine entscheidende Rolle, jedoch sollten wegen der schwierigen Handhabung zu große und zu kleine Teilchen vermieden werden. Im allgemeinen sind Teilchengrößen von 5 bis 1000 µ erstrebenswert, solche von 20 bis 200 µ bevorzugt. Die Teilchen können von gleicher Größe sein (homogene Korngrößenverteilung), sie können jedoch auch unterschiedliche Größen haben, wobei ein Korngrößenverteilungsspektrum innerhalb des genannten Bereiches erstrebenswert ist.

Die Menge des den Hydroxy-benzotriazolen oder Hydroxy-oxo-dihydro-benzotriazinen zuzumischenden Phlegmatisierungsmittels ist nach oben unbegrenzt, jedoch sollte nicht wesentlich mehr Feststoff hinzugefügt werden, als zur Erreichung des gewünschten Effekts erforderlich ist. Demgemäß empfiehlt es sich, nicht mehr als 100 bis ca. 200 Gew.-% des Phlegmatisierungsmittels, bezogen auf das in fester Form vorliegende Hydroxy-benzotriazol oder Hydroxy-oxo-dihydro-benzotriazin anzuwenden. Als untere Grenze sollte das Gemisch 50—60 Gew.-% Phlegmatisierungsmittel (bezogen auf das reine Hydroxy-benzotriazol bzw. Hydroxy-oxo-dihydro-benzotriazin) enthalten. Bevorzugt ist ein Gehalt von 50—80 Gew.-% Phlegmatisierungsmittel, bezogen auf das trockene Gemisch.

Sofern die Hydroxy-benzotriazole oder Hydroxy-oxo-dihydro-benzotriazine für Peptidsynthesen eingesetzt werden, stören die zugesetzten Phlegmatisierungsmittel die Reaktion nicht. Man erhält die Peptide in den gleichen Ausbeuten und in derselben Reinheit wie bei Umsetzungen in Abwesenheit eines Phlegmatisierungsmittels.

Die Abtrennung des unlöslichen Phlegmatisierungsmittels erfolgt bei diesen Synthesen zweckmäßigerweise nach der Umsetzung zusammen mit dem ebenfalls schwer löslichen Nebenprodukt Dicyclohexylharnstoff. Wie üblich kann diese Abtrennung beispielsweise durch Filtration oder Zentrifugieren vorgenommen werden. Man kann das Phlegmatisierungsmittel aber auch vor der Reaktion abtrennen. Dazu suspendiert man das ein Phlegmatisierungsmittel enthaltende Hydroxy-benzotriazol bzw.

Hydroxy-oxo-dihydro-benzotriazin in einem Lösungsmittel, in dem dieses löslich, das Phlegmatisierungsmittel jedoch unlöslich ist, filtriert vom Ungelösten ab oder trennt die Lösung auf andere Weise vom Ungelösten und verwendet das Filtrat zur Umsetzung. Es empfiehlt sich, in diesem Falle ein Lösungsmittel zu verwenden, in dem auch die anschließende Peptidsynthese durchgeführt werden kann, z. B. Dimethylformamid oder Dimethylacetamid.

Die phlegmatisierten Gemische aus Hydroxy-benzotriazol oder Hydroxy-oxo-dihydro-benzotriazin und dem Phlegmatisierungsmittel kann man durch Vermischen der Substanzen gewinnen. Vorteilhaft kann dieses Vermischen in Gegenwart einer geeigneten Flüssigkeit geschehen. So kann man beispielsweise Hydroxy-benzotriazol oder Hydroxy-oxo-dihydro-benzotriazin in einem Lösungsmitel wie Methanol oder Dimethylformamid auflösen, unter Rühren das Phlegmatisierungsmittel zusetzen und mit einem Fällungsmittel, z. B. Wasser, ausfällen. Man kann aber auch das Phlegmatisierungsmittel im Fällungsmittel vorlegen und unter Rühren die Lösung des Hydroxy-benzotriazols bzw. Hydroxy-oxo-dihydro-benzotriazins in einem Lösungsmittel hinzufügen.

Die erhaltenen Gemische enthalten das Hydroxy-benzotriazol bzw. Hydroxy-oxo-dihydro-benzotriazin einerseits und das Phlegmatisierungsmittel andererseits in gleichmäßiger (homogener) Verteilung. Falls die Verteilung aus besonderen Gründen nicht völlig homogen sein sollte, kann man die Gemische in einer geeigneten Flüssigkeit, z. B. Petroläther, verrühren und von dieser abtrennen. Dadurch werden sie vollständig homogenisiert.

Das erfindungsgemäße Verfahren ermöglicht es, Hydroxybenzotriazole bzw. Hydroxy-oxo-dihydro-benzotriazine gefahrlos zu handhaben und insbesondere bei Peptidsynthesen nach der Dicyclohexylcarbodiimid- oder der Aktivester-Methode gefahrlos einzusetzen. Die Beseitigung der Explosionsgefahr ist insbesondere für Synthesen in großem Maßstab von Bedeutung.

Die beigefügte Tabelle gibt einige Daten zur Explosionsgefährlichkeit von Hydroxy-benzotriazolen und Hydroxy-oxo-dihydro-benzotriazinen sowie deren Mischungen mit Phlegmatisierungsmitteln.

Durch die nachstehenden Beispiele wird die Erfindung näher erläutert.

Tabelle

Explosionsgefährlichkeit von Hydroxy-benzotriazolen und Hydroxy-oxo-dihydro-benzotriazinen sowie deren Mischungen mit Phlegmatisierungsmitteln

| Unter-suchte Substanz | Phlegma-tisierungs-mittel | Anteil Phleg-matisierungs-mittel in Gewichts-prozent | Schlagempfind-lichkeit (BAM-Fallhammer) Fallgewicht × Fallhöhe | Ergebnis des Stahlhülsentests: (Koenen-Test) Grenzdurchmesser der Düse (mm) | explosions-gefährlich ja/nein |
|---|---|---|---|---|---|
| HOBT*) | — | 0 | 5 kg × 50 cm | 12 | ja |
| HOOBT**) | — | 0 | 5 kg × 10 cm | 6 | ja |
| HOOBT | Wasser | 60 | bis 10 kg × 100 cm keine Explosion | 2 | ja |
| HOOBT | Dicyclohexyl-harnstoff | 50 | 10 kg × 70 cm | bei 1 keine Explosion | nein |
| HOBT | Filterhilfs-mittel***) | 55 | bis 10 kg × 100 cm keine Explosion | 1 | nein |
| HOBT | Aktivkohle****) | 60 | bis 10 kg × 100 cm keine Explosion | bei 1 keine Explosion | nein |
| HOOBT | Filterhilfs-mittel***) | 60 | 5 kg × 90 cm | bei 1 keine Explosion | nein |

*) 1-Hydroxy-benzotriazol
**) 3-Hydroxy-4-oxo-3,4-dihydro-1,2,3-benzotriazin
***) als Filterhilfsmittel wurde Kieselgur Seitz-Spezial der Firma Seitz-Filter-Werke, Bad Kreuznach verwendet.
****) als Aktivkohle wurde Aktivkohle der Fa. Riedel de Häen, Seelze-Hannover benutzt.

## Beispiel 1

### Herstellung des Gemisches 1-Hydroxy-benzotriazol/Filterhilfsmittel Seitz-Spezial

Es werden in 59 g Äthanol 23,7 g 2-Chlor-nitrobenzol und 27 g Hydrazinhydrat (80%ig) unter Rühren gelöst. Es wird ca. 15 Stunden am Rückfluß gekocht und anschließend nach dem Abkühlen auf Raumtemperatur mit 375 ml Wasser versetzt. Vom Niederschlag wird abfiltriert und das Filtrat unter Kühlung mit ca. 29,5 g konzentrierter Salzsäure auf pH 1 gestellt. Der abfiltrierte Niederschlag wird unter schwachem Erwärmen in ca. 87 ml Methanol gelöst und über Aktivkohle filtriert.

Es werden 1,4 l Wasser unter Rühren mit 24 g Seitz-Spezial (Kieselgur) versetzt. Unter gutem Rührem wird das obige Filtrat zugegeben. Nach Abkühlen auf ca. 0—5"C wird filtriert und das erhaltene Produkt getrocknet.

Der Gehalt an 1-Hydroxy-benzotriazol im Gemisch wird durch Titration mit Natronlauge bestimmt. Es werden mehrere Proben analysiert.

Ausbeute: 30,5 g Gemisch.
Gehalt an 1-Hydroxy-benzotriazol 25%.

Falls das Gemisch nicht genügend homogen ist, wird es durch Verrühren in Petroläther homogenisiert.

## Beispiel 2

### Herstellung des Gemisches
### 3-Hydroxy-4-oxo-3,4-dihydro-1,2,3-benzotriazin/Filterhilfsmittel Seitz-Spezial

Es werden unter Rühren 5,316 g Anthranilhydroxamsäure-Natriumsalz in 28,5 ml Wasser gelöst. Unter Kühlung und Rühren werden bei ca. 1—5°C 17 ml halbkonzentrierte Salzsäure und anschließend die Lösung von 2,21 g Natriumnitrit in 15,5 ml Wasser zugegeben.

Nach 30 minütigem Nachrühren wird abfiltriert und mit 50 ml Wasser gewaschen. Das ungetrocknete Produkt wird in 17 ml Dimethylformamid weitgehend gelöst. Nach der Zugabe von 75 ml Wasser wird 30 Minuten gerührt, abfiltriert und mit 50 ml Wasser gewaschen. Das ungetrocknete Produkt wird unter leichtem Erwärmen in 15 ml Dimethylformamid gelöst und unter Rühren mit 6,63 g Seitz-Spezial (Kieselgur) versetzt. Es wird 30 Minuten nachgerührt. Bei ca. 5°C werden 235 ml Wasser zugegeben, nach Nachrühren wird abfiltriert, mit Wasser gewaschen und getrocknet.

Der Gehalt an 3-Hydroxy-4-oxo-3,4-dihydro-1,2,3-benzotriazin im Gemisch wird durch Titration mit Natronlauge bestimmt. Es werden mehrere Proben analysiert.

Ausbeute 8,2 g
Gehalt an 3-Hydroxy-4-oxo-3,4-dihydro-1,2,3-benzotriazin 26%.

Falls das Gemisch nicht genügend homogen ist, wird es durch Verrühren in Petroläther homogenisiert.

## Beispiel 3

### Herstellung des Gemisches 1-Hydroxy-benzotriazol/Aktivkohle

Es werden unter Rühren 100 g 1-Hydroxy-benzotriazol in 200 ml Dimethylformamid gelöst. Nach der Zugabe von 100 g Aktivkohle werden unter Rühren und Kühlen 4 l Wasser zugegeben. Es wird 1 Stunde nachgerührt, anschließend abgesaugt und getrocknet.

Ausbeute 167 g Gemisch.
Gehalt an 1-Hydroxy-benzotriazol 40%.

## Beispiel 4

### Herstellung des Gemisches
### 3-Hydroxy-4-oxo-3,4-dihydro-1,2,3-benzotriazin/Dicyclohexylharnstoff

Es werden unter Rühren 110 g 3-Hydroxy-4-oxo-3,4-dihydro-1,2,3-benzotriazin in 240 ml Dimethylformamid bei 30°C gelöst. Nach der Zugabe von 90 g Dicyclohexylharnstoff werden unter

0 064 714

Rühren und Kühlen 4 l Wasser zugefügt. Nach kurzem Nachrühren bei 3°C wird abgesaugt und getrocknet.

Ausbeute 180 g Gemisch.
Gehalt an 3-Hydroxy-4-oxo-3,4-dihydro-1,2,3-benzotriazin 50%.

## Beispiel 5

### Herstellung von Z-Ser(tBu)-Glu(OtBu)-Leu-OMe nach der Dicyclohexylcarbodiimid-Methode in Gegenwart von 1-Hydroxy-benzotriazol/Filterhilfsmittel-Gemisch

Die Lösung von 3,77 kg Z-Ser(tBu)-OH und 4,681 kg H-Glu(OtBu)-Leu-OMe × HCl in 15,5 l Dimethylformamid wird unter Rühren mit 5,189 kg 1-Hydroxy-benzotriazol/Filterhilfsmittel Seitz-Spezial (Kieselgur)-Gemisch (Gehalt an 1-Hydroxy-benzotriazol 33,2%) versetzt.

Nach dem Abkühlen auf 0°C wird die Lösung von 2,628 kg Dicyclohexylcarbodiimid und 1,633 l N-Ethylmorpholin in 8,47 l Dimethylformamid zugegeben. Es wird 16 Stunden bei 0°C gerührt, vom ungelösten Dicyclohexylharnstoff und Filterhilfsmittel abfiltriert und das Filtrat im Vakuum eingeengt. Der Rückstand wird in 60 l Ethylacetat aufgenommen, die Ethylacetat-Lösung mit wäßriger Natriumhydrogencarbonat- und Kaliumhydrogensulfat-Lösung sowie mit Wasser extrahiert, über Natriumsulfat getrocknet, filtriert und schließlich im Vakuum auf 30 l eingedampft. Es werden 200 l Petroläther zugegeben und das Produkt abfiltriert und getrocknet.

Ausbeute 5,67 kg (73,1% d. Th.)
Schmp. 132—134°C $[\alpha]_D$: $-22°$ (c = 1 in Methanol).

Bei der Herstellung des gleichen Peptides nach der selben Methode in Abwesenheit von Filterhilfsmittel werden vergleichbare Werte erhalten:

Ausbeute 74% d. Th.,
Schmp. 133—134°C $[\alpha]_D$: $-24,1°$ (c = 1 in Methanol), s. G. Jäger, W. König, H. Wissmann und R. Geiger, Chem. Ber. Band 107, Seiten 215—231 (1974).

## Beispiel 6

### Herstellung von Z₃-Arg-Leu-GIN-Arg-Leu-Leu-GIN-Gly-Leu-Val-NH₂ × HClO₄ nach der Dicyclohexylcarbodiimid-Methode in Gegenwart von 3-Hydroxy-4-oxo-3,4-dihydro-1,2,3-benzotriazin/Filterhilfsmittel-Gemisch

Die Lösung von 1,374 kg Z₃-Arg-Leu-GIN-OH und 1,912 kg H-Arg-Leu-Leu-GIN-Gly-Leu-Val-NH₂ × 2 HClO₄ in 11,8 l Dimethylacetamid wird unter Rühren mit 1,066 kg 3-Hydroxy-4-oxo-3,4-dihydro-1,2,3-benzotriazin/Filterhilfsmittel Seitz-Spezial (Kieselgur)-Gemisch (Gehalt an 3-Hydroxy-4-oxo-3,4-dihydro-1,2,3-benzotriazin 25,7%) versetzt. Nach der Zugabe von 215 ml N-Ethylmorpholin und der Lösung von 382 g Dicyclohexylcarbodiimid in 5 l Dimethylacetamid wird 18 Stunden gerührt und dann vom ungelösten Dicyclohexylharnstoff und Filterhilfsmittel abgesaugt. Das Filtrat wird in ein Gemisch aus 101 l Eiswasser und 2,53 l 2nHClO₄ bei 2°C eingetragen und das ausgefallene Produkt abfiltriert. Es wird mit Wasser gewaschen und aus 10 l Dimethylacetamid und 100 l Ethylacetat umgefällt.

Ausbeute 2,43 kg (75% d. Th.)
Schmp. 241°C $[\alpha]_D$: $-32°$ (c = 1 in 80%iger Essigsäure).

Bei der Herstellung in Abwesenheit von Filterhilfsmittel werden für das Dekapeptid-hydrobromid folgende Werte erhalten:

Ausbeute 78;
$[\alpha]_D$: $-35°$ (c = 1 in 80%iger Essigsäure); s. G. Jäger, W. König, H.Wissmann und R. Geiger, Chem. Ber. Band 107, Seiten 215—231 (1974).

5

**0 064 714**

Beispiel 7

Herstellung von $Z_3$-Arg-Asp(OtBu)-Ser(tBu)-Ala-OH nach der Aktivester-Methode in Gegenwart von
1-Hydroxybenzotriazol/Filterhilfsmittel-Gemisch

1,002 kg H-Asp(OtBu)-Ser(tBu)-Ala-OH werden in 5 l Dimethylformamid gelöst. 0,908 kg 1-Hydroxy-benzotriazol/Filterhilfsmittel Seitz-Spezial-Gemisch (Gehalt an 1-Hydroxy-benzotriazol 37%) werden in 2 l Dimethylformamid suspendiert. Nach Rühren wird vom ungelösten Filterhilfsmittel abfiltriert und das Filtrat mit obiger Lösung vereinigt. In die Lösung werden anschließend 1,877 kg $Z_3$-Arg-OTcp eingetragen und 18 Stunden bei Raumtemperatur gerührt. Die Lösung wird unter Rühren in 63 l eiskaltes Wasser einlaufen gelassen und der erhaltene Niederschlag abfiltriert und getrocknet. Das zerkleinerte Rohprodukt wird mit 40 l Ethylacetat ausgekocht.

Ausbeute 1,77 kg (74% d. Th.)
Schmp. 171°C; $[\alpha]_D$: −5,9° (c = 1 in Eisessig)

Bei der Herstellung des gleichen Peptids mit $Z_3$-Arg-ONp in Abwesenheit eines Phlegmatisierungsmittels wurden vergleichbare Werte erzielt: Ausbeute 86%, Schmp. 172−173°C und $[\alpha]_D$: −6,6° (s. E. Wünsch, G. Wendlberger und P. Thamm, Chem. Ber. Band 104, Seiten 2445−2453, 1971).

## Patentansprüche

1. Verfahren zur Phlegmatisierung von Hydroxy-benzotriazolen oder Hydroxy-oxo-dihydro-benzotriazinen, dadurch gekennzeichnet, daß man diesen als Phlegmatisierungsmittel feinverteilte inerte Feststoffe zusetzt.

2. Verfahren nach Anspruch 1, dadurch gekennzeichnet, daß als Phlegmatisierungsmittel Filterhilfsmittel auf Silikatbasis oder Aktivkohle verwendet wird.

3. Verfahren nach Anspruch 1, dadurch gekennzeichnet, daß als Phlegmatisierungsmittel Dicyclohexylharnstoff verwendet wird.

4. Verfahren nach einem der Ansprüche 1 bis 3, dadurch gekennzeichnet, daß das Phlegmatisierungsmittel eine Korngröße von 5 bis 1000 µ hat.

5. Verfahren gemäß einem der Ansprüche 1 bis 4, dadurch gekennzeichnet, daß man 50 bis 80 Gewichtsprozent Phlegmatisierungsmittel (bezogen auf die Gesamtmenge des Gemisches) zusetzt.

6. Mittel enthaltend

(A) ein Hydroxy-benzotriazol oder ein Hydroxy-oxo-dihydrobenzotriazin und
(B) einen feinverteilten inerten Feststoff.

7. Mittel gemäß Anspruch 6, dadurch gekennzeichnet, daß es in fester Form vorliegt.

8. Mittel gemäß Anspruch 6 oder 7, dadurch gekennzeichnet, daß als Komponente B Filterhilfsmittel auf Silikatbasis oder Aktivkohle verwendet wird.

9. Mittel gemäß Anspruch 6 oder 7, dadurch gekennzeichnet, daß als Komponente B Dicyclohexylharnstoff verwendet wird.

10. Mittel gemäß einem der Ansprüche 6 bis 9, dadurch gekennzeichnet, daß die Komponente B eine Korngröße von 5 bis 1000 µ hat.

11. Mittel gemäß einem der Ansprüche 6 bis 10, dadurch gekennzeichnet, daß die Menge an der Komponente B im Hydroxy-benzotriazol- bzw. Hydroxy-oxo-dihydrobenzotriazin-Gemisch 50−80 Gewichtsprozent beträgt.

12. Verwendung feinverteilter inerter Feststoffe zur Phlegmatisierung von Hydroxy-benzotriazolen bzw. Hydroxy-oxo-dihydrobenzotriazinen.

## Claims

1. A process for desensitizing a hydroxybenzotriazole or a hydroxyoxodihydrobenzotriazine, which comprises adding finely divided inert solids to the latter as a desensitizing agent.

2. The process as claimed in claim 1, characterized in that a filter aid based on silicates, or active charcoal is used as the desensitizing agent.

3. The process as claimed in claim 1, characterized in that dicyclohexylurea is used as the desensitiz-ing agent.

4. The process as claimed in any of claims 1 to 3, characterized in that the desensitizing agent has a particle size of 5 to 1000 µ.

5. The process as claimed in any of claims 1 to 4, characterized in that 50 − 80 per cent by weight of the desensitizing agent (related to the total quantity of the mixture) are added.

6

6. A composition containing

(A) a hydroxybenzotriazole or a hydroxyoxodihydrobenzotriazine and
(B) a finely divided inert solid.

7. A composition as claimed in claim 6 in the form of a solid.

8. A composition as claimed in claim 6 or 7, characterized in that a filter aid based on silicates, or active charcoal is used as the component B.

9. A composition as claimed in claim 6 or 7, characterized in that dicyclohexylurea is used as the component B.

10. A composition as claimed in any of claims 6 to 9, wherein the component B has a particle size of 5 to 1000 μ.

11. A composition as claimed in any of claims 6 to 10, wherein the quantity of the component B in the hydroxybenzotriazole or hydroxyoxodihydrobenzotriazine mixture is 50—80 per cent by weight.

12. The use of finely divided inert solids for desensitizing hydroxybenzotriazoles or hydroxyoxodihydrobenzotriazines.

## Revendications

1. Procédé de phlegmatisation d'hydroxy-benzotriazoles ou d'hydroxy-oxo-dihydro-benzotriazines, caractérisé en ce qu'on ajoute à ces produits des substances solides inertes, finement divisées, en tant qu'agent de phlegmatisation.

2. Procédé selon la revendication 1, caractérisé en ce qu'on utilise un adjuvant de filtration à base de silicate ou du charbon actif en tant qu'agent de phlegmatisation.

3. Procédé selon la revendication 1, caractérisé en ce qu'on utilise la dicyclohexylurée en tant qu'agent de phlegmatisation.

4. Procédé selon l'une quelconque des revendications 1 à 3, caractérisé en ce que l'agent de phlegmatisation a une grosseur de particules de 5 à 1000 μm.

5. Procédé selon l'une quelconque des revendications 1 à 4, caractérisé en ce qu'on ajoute de 50 à 80% en poids d'agent de phlegmatisation (par rapport à la masse totale du mélange).

6. Agent contenant:

(A) un hydroxy-benzotriazole ou une hydroxy-oxo-dihydrobenzotriazine et
(B) une substance solide inerte finement divisée.

7. Agent selon la revendication 6, caractérisé en ce qu'il se présente sous une forme solide.

8. Agent selon la revendication 6 ou 7, caractérisé en ce qu'on utilise un adjuvant de filtration à base de silicate, ou du charbon actif en tant que constituant B.

9. Agent selon la revendication 6 ou 7, caractérisé en ce qu'on utilise de la dicyclohexylurée en tant que constituant B.

10. Agent selon l'une des revendications 6 à 9, caractérisé en ce que le constituant B à une grosseur de particules de 5 à 1000 μm.

11. Agent selon l'une des revendications 6 à 10, caractérisé en ce que la quantité du constituant B dans le mélange d'hydroxy-benzotriazole ou d'hydroxy-oxo-dihydrobenzo-triazine est de 50 à 80% en poids.

12. Utilisation de substances solides inertes, finement divisées, pour la phlegmatisation d'hydroxy-benzotriazoles ou d'hydroxy-oxo-dihydro-benzotriazines.